# FASCICULE DE BREVET EUROPEEN

(11) **EP 3 274 705 B1**
(45) Date de publication et mention de la délivrance du brevet: **13.03.2019**
(21) Numéro de dépôt: 16711249.9
(22) Date de dépôt: 18.03.2016
(51) Int. Cl.: G01N 33/46, G01N 27/04

(54) **DISPOSITIF DE METROLOGIE ET PROCEDE ASSOCIE**
METROLOGIEVORRICHTUNG UND ZUGEHÖRIGES VERFAHREN
METROLOGY DEVICE AND ASSOCIATED METHOD

(30) Priorité: 24.03.2015 FR 1552471
(43) Date de publication de la demande: 31.01.2018
(73) Titulaire: Tecsan, 1950 Sion 2 (CH)
(72) Inventeur: SANDOZ, Jean-Luc, 1024 Ecublens (CH); BENNOIT, Yann, 1025 Saint-Sulpice (CH); GASSER, Jean-Daniel, 1030 Bussigny (CH)
(74) Mandataire: Cabinet Laurent & Charras
(86) Numéro de dépôt international: PCT/EP2016/056057
(87) Numéro de publication internationale: WO 2016/150882

(56) Documents cités:
- NL-A- 9 101 010
- EZER E ED - INSTITUTE OF ELECTRICAL AND ELECTRONICS ENGINEERS: "Measurement of wood pole strength-Polux<(R)> a new nondestructive inspection method", 2001 RURAL ELECTRIC POWER CONFERENCE. LITTLE ROCK, AR, APRIL 29 - MAY 1, 2001; [RURAL ELECTRIC POWER CONFERENCE], NEW YORK, NY : IEEE, US, 29 avril 2001 (2001-04-29), pages C6/1-C6/7, XP010556184, ISBN: 978-0-7803-7012-8

## Description

### DOMAINE TECHNIQUE

La présente invention concerne un dispositif de métrologie pour déterminer la qualité d'un matériau hygroscopique, tel que du bois ou autres matériaux de construction analogues. La présente invention concerne également le procédé associé.

L'invention vise plus particulièrement à déterminer la qualité des poteaux en bois utilisés autant par les électriciens pour la distribution et le transport de l'énergie que par les organismes de télécommunication pour les réseaux câblés ou pour les réseaux de fibres optiques aériens.

### ART ANTERIEUR

Il existe dans le monde des millions de poteaux en bois en service, qu'il faut périodiquement contrôler pour assurer la maintenance des lignes pour deux raisons principales. Premièrement, la qualité des poteaux en bois engage la sécurité des personnes évoluant à côté de ces poteaux et les pouvoirs publics imposent souvent une maintenance préventive de ces infrastructures. Deuxièmement, la connaissance de la qualité des poteaux en bois permet d'anticiper les remplacements à venir sur le réseau.

Pendant de très longues années, les poteaux de bois ont été visités par des hommes d'expérience qui frappaient le poteau avec un marteau pour écouter ses propriétés de résonnance. Cette méthode, bien qu'empirique, donnait des résultats relativement satisfaisants et en tout cas supérieurs aux réseaux où aucune visite de contrôle n'était effectuée.

Depuis quelques décennies, des technologies non destructives basées sur des mesures physiques du bois sont apparues.

Les brevets US 2,389,030 et US 4,343,179 décrivent une technologie de type résistographe, liée à la densité du bois, qui consiste à faire pénétrer un élément dans le bois et à mesurer l'effort nécessaire à la pénétration de l'élément. Dans le cas du premier document, un support est fixé au poteau au moyen de deux pointes de faible longueur et d'un cerclage autour du poteau. Une mèche métallique, guidée par le support, est visée par un dispositif manuel dans le bois et la résistance de vissage de la mèche est mesurée. Dans le cas du second document, un opérateur maintien le dispositif contre le poteau et provoque une pénétration linéaire d'une pointe par un système dynamométrique. Lorsque la pointe pénètre dans le poteau, la résistance de pénétration de la pointe est mesurée par une règle.

Ces deux méthodes permettent d'obtenir un densitogramme sur lequel il est possible d'observer des poches de pourriture ou des poches de faible densité à l'intérieur même du poteau.

En revanche, ces méthodes ne délivrent aucun diagnostic direct sur la performance structurelle résiduelle du poteau et donc sur son niveau de sécurité. Elles se limitent à montrer qu'il existe une dégradation biologique plus ou moins importante à l'intérieur du poteau. En outre, ces méthodes ne détectent pas des poteaux sans pourriture, mais qui pourraient contenir seulement des fibres relativement faibles et qui s'avéreraient par conséquent très faibles en performance mécanique, et pouvant représenter un risque. De même, ces méthodes sont incapables de donner une quelconque estimation du temps pour lequel le poteau pourrait encore rester en service. C'est une méthode assez binaire, indiquant soit que le poteau contient une dégradation, soit qu'il n'en contient pas.

Le brevet FR 2 707 759 permet de surmonter ces obstacles en proposant une méthode visant à quantifier une valeur résiduelle de performance en flexion ainsi qu'une espérance de durée de vie estimée du poteau. Cette méthode consiste à fixer un cadre au poteau par deux éléments de support de type sangle. Deux pointes sont déplacées manuellement en translation par rapport au cadre pour pénétrer dans le poteau et permettent de mesurer à la fois un effort de pénétration de chaque pointe dans le poteau et une résistivité électrique entre les extrémités des deux pointes lorsque les deux pointes sont insérées dans le poteau. La résistivité électrique entre les deux pointes du poteau permet de rendre compte de l'hygroscopie du bois en profondeur à l'intérieure du poteau.

Cependant, cette méthode présente l'inconvénient d'être assez longue à mettre en oeuvre car un opérateur doit fixer solidement les deux éléments de support de type sangle avant de procédé à la première mesure.

Le document EZER E.: "Measurement of wood pole strength-Polux a new nondestructive inspection method",2001 RURAL ELECTRIC POWER CONFERENCE. LITTLE ROCK, AR, APRIL 29 - MAY 1, 2001; [RURAL ELECTRIC POWER CONFERENCE], NEW YORK, NY : IEEE, US, 29 avril 2001 (2001-04-29), pages C6/1-C6/7, XP010556184, ISBN: 978-0-7803-7012-8
et le brevet NL9101010A décrivent aussi des appareils combinant une mesure mécanique et une mesure électrique pour déterminer la qualité d'un poteau en bois.

### EXPOSE DE L'INVENTION

La présente invention vie à améliorer la précision des dispositifs existants au moyen d'une troisième mesure complémentaire correspondant à un effort de pénétration d'un élément de support dans le poteau. L'étape de fixation du cadre par un opérateur devient ainsi une première étape de mesure.

Selon un premier aspect, l'invention concerne un dispositif de métrologie de la qualité d'un matériau hygroscopique, tel que du bois ou autres matériaux de construction analogues, comprenant :
- un cadre,
- un élément de support apte à fixer le cadre au matériau hygroscopique,
- deux pointes mobiles en translation par rapport au cadre,
- des moyens de mesure d'un effort de pénétration de chaque pointe en fonction d'une force appliquée sur chaque pointe pour atteindre une distance prédéterminée,
- des moyens de mesure d'une résistivité électrique entre les extrémités des deux pointes aptes à mesurer une résistivité électrique du matériau hygroscopique lorsque les deux pointes sont insérées dans le matériau hygroscopique, et
- des moyens de mesure d'un effort de pénétration de l'élément de support en fonction d'une force appliquée sur l'élément de support pour atteindre une position de fixation prédéterminée, l'élément de support étant apte à pénétrer dans le matériau hygroscopique pour fixer le cadre au matériau hygroscopique.

L'invention permet ainsi d'améliorer la précision des mesures effectuées sur les matériaux hygroscopique, notamment les poteaux de bois. La fixation du cadre au matériau hygroscopique n'est plus une perte de temps pour l'opérateur car une première mesure peut directement être effectuée pendant cette phase.

Contrairement aux dispositifs maintenus par un cerclage autour du poteau, l'invention permet d'éviter de nettoyer la base du poteau pour positionner un cerclage. En outre, certains poteaux sont positionnés contre un mur ou le long d'un trottoir empêchant de positionner un cerclage à la base du poteau. Une gaine peut également être positionnée le long du poteau. Dans ce cas, le serrage du cerclage risque d'endommager cette gaine. L'invention permet également de surmonter ces inconvénients.

Selon un mode de réalisation, le dispositif comporte :
- au moins un moyen de déplacement motorisé des pointes et/ou de l'élément de support, ledit moyen de déplacement étant commandé par une commande de déplacement, et
- au moins un moyen de régulation de la commande de déplacement.

Les dispositifs de l'état de la technique sont mis en oeuvres mécaniquement par un opérateur dont les efforts varient entre deux manipulations. Ce mode de réalisation améliore la précision des mesures effectuées en régulant la précision des déplacements des outils.

Selon un mode de réalisation, le dispositif comporte au moins une source d'énergie autonome. Ce mode de réalisation permet au dispositif d'être autonome en énergie et portatif.

Selon un mode de réalisation, le dispositif comporte une pièce de guidage de la position du cadre par rapport au matériau hygroscopique, la pièce de guidage comportant deux pointes aptes à être insérées dans le matériau hygroscopique sur quelques millimètres. Ce mode de réalisation améliore la précision de la première mesure effectuée lors du vissage de l'élément de support, car la position du dispositif est guidée.

Selon un mode de réalisation, les pointes et/ou l'élément de support sont montés sur des éléments de fixation amovible par rapport au cadre. Ce mode de réalisation permet de changer facilement les pointes et/ou l'élément de support lors d'une opération de maintenance ou d'adaptation des outils au matériau hygroscopique.

Selon un mode de réalisation, le dispositif comporte :
- des moyens de stockage des valeurs des efforts de pénétration de chaque pointe, de la résistivité électrique et de l'effort de pénétration de l'élément de support, et
- des moyens de traitement aptes à estimer un état de sécurité et une durée de vie résiduelle du matériau hygroscopique en fonction des valeurs des efforts de pénétration de chaque pointe, de la résistivité électrique et de l'effort de pénétration de l'élément de support.

Ce mode de réalisation permet de délivrer rapidement pour l'opérateur une estimation de l'état du matériau hygroscopique.

Selon un mode de réalisation, le dispositif comporte :
- des moyens d'émission disposés à l'intérieur du cadre aptes à transmettre les valeurs des efforts de pénétration de chaque pointe, la résistivité électrique et l'effort de pénétration de l'élément de support, et
- un calculateur de terrain distant par rapport au cadre comportant :
   - des moyens de réception aptes à recevoir les informations des moyens d'émission disposés à l'intérieur du cadre,
   - des moyens de stockage des paramètres du matériau hygroscopique, et
   - des moyens de traitement intégrés dans le calculateur de terrain aptes à estimer un état de sécurité et une durée de vie résiduelle du matériau hygroscopique en fonction des valeurs des efforts de pénétration de chaque pointe, de la résistivité électrique, de l'effort de pénétration de l'élément de support, et des paramètres du matériau hygroscopique.

Ce mode de réalisation permet de centraliser plusieurs mesures de plusieurs matériaux hygroscopiques et d'obtenir une estimation globale d'un parc de matériaux hygroscopiques.

Selon un mode de réalisation, les paramètres du matériau hygroscopique comportent l'essence du matériau hygroscopique, le diamètre, le type de traitement chimique subit par le matériau hygroscopique, et la longueur hors sol du matériau hygroscopique. Ce mode de réalisation améliore la précision des estimations sur l'état de santé du matériau hygroscopique.

Selon un mode de réalisation, la distance prédéterminée d'enfoncement des pointes est comprise entre 40 et 60mm. Ce mode de réalisation permet d'effectuer les mesures au coeur du matériau hygroscopique.

Selon un mode de réalisation, l'élément de support est une vis à bois dont le diamètre est adapté à l'essence et au diamètre du matériau hygroscopique. Ce mode de réalisation améliore la solidité de la fixation entre le cadre et le matériau hygroscopique.

Selon un deuxième aspect, l'invention concerne un procédé de métrologie de la qualité d'un matériau hygroscopique, tel que du bois ou d'autres matériaux de construction analogues, comprenant les étapes consistant à :
- insérer deux pointes dans le matériau hygroscopique jusqu'à une distance prédéterminée,
- mesurer, pour chaque pointe, un effort de pénétration en fonction de la force appliquée sur chaque pointe pour atteindre la distance prédéterminée,
- lorsque les deux pointes ont atteints la distance prédéterminée, mesurer une résistivité électrique entre les extrémités des deux pointes insérée dans le matériau hygroscopique,
- estimer une hygrométrie interne du matériau hygroscopique en fonction de la résistivité électrique,
- insérer un élément de support dans le matériau hygroscopique suivant un angle par rapport au cadre sensiblement égal à l'angle de pénétration des pointes par rapport au cadre,
- mesurer un effort de pénétration appliquée sur l'élément de support pour atteindre une position de fixation prédéterminée, et
- estimer un état de sécurité et une durée de vie résiduelle du matériau hygroscopique en fonction des valeurs des efforts de pénétration de chaque pointe, de la résistivité électrique et de l'effort de pénétration de l'élément de support.

L'angle de l'élément de support par rapport au cadre étant sensiblement égal à l'angle des pointes par rapport au cadre, la force exercée sur le cadre lors de l'insertion des pointes dans le matériau hygroscopique présente une direction opposée à la force de maintien du cadre par l'élément de support. Ce deuxième aspect de l'invention permet ainsi d'orienter angulairement le dispositif par rapport à la hauteur du matériau hygroscopique en limitant les risques d'arrachage du dispositif. L'invention permet également d'insérer les pointes dans le matériau hygroscopique sur une plus grande distance sans traverser le matériau hygroscopique.

Selon un mode de réalisation, le matériau hygroscopique étant planté sur un sol, l'étape consistant à insérer un élément de support dans le matériau hygroscopique est effectuée au niveau du sol. Ce mode de réalisation permet d'insérer les pointes dans le matériau hygroscopique sous le niveau du sol dans une zone particulièrement propice à la formation des pourritures du matériau hygroscopique.

Selon un mode de réalisation, l'élément de support pénètre dans le matériau hygroscopique suivant un angle par rapport au sol sensiblement égal à l'angle de pénétration des pointes par rapport au sol.

Selon un mode de réalisation, l'élément de support et les deux pointes dans le matériau hygroscopique sont effectuées avec un angle compris entre 30 et 45 degrés par rapport au sol.

Selon un mode de réalisation, le matériau hygroscopique étant planté sur un sol, l'étape consistant à insérer un élément de support dans le matériau hygroscopique est effectuée avec un angle compris entre 30 et 45 degrés par rapport au sol de sorte que l'élément de support pénètre sous le niveau du sol.

### DESCRIPTION SOMMAIRE DES FIGURES

La manière de réaliser l'invention ainsi que les avantages qui en découlent, ressortiront bien du mode de réalisation qui suit, donné à titre indicatif mais non limitatif, à l'appui des figures annexées dans lesquelles les figures 1 à 5 représentent :
- Figure 1 : une représentation schématique en coupe latérale d'un dispositif de métrologie de la qualité d'un poteau ;
- Figure 2 : une représentation schématique en coupe horizontale du dispositif de la Figure 1 dans une première étape ;
- Figure 3 : une représentation schématique en coupe horizontale du dispositif de la Figure 1 dans une deuxième étape ;
- Figure 4 : une représentation schématique en coupe horizontale du dispositif de la Figure 1 dans une troisième étape ; et
- Figure 5 : un organigramme des étapes d'un procédé de métrologie à l'aide du dispositif de la Figure 1.

### DESCRIPTION DETAILLEE DE L'INVENTION

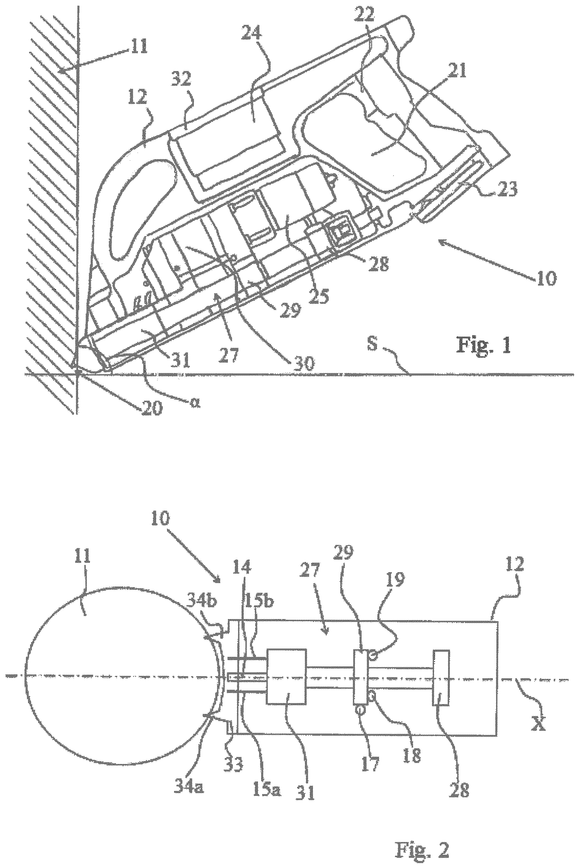

Le mode de réalisation des Figures 1 à 4 illustre un dispositif de métrologie 10 de la qualité d'un poteau en bois 11. Le poteau en bois 11 est inséré dans un sol S avec un angle sensiblement égal à 90° entre la hauteur du poteau 11 et le sol S.

Le dispositif 10 comporte un cadre 12 intégrant une poignée 21 à l'intérieur de laquelle est agencée une gâchette électrique 22. La gâchette 22 est alimentée par un circuit d'alimentation 23 connecté à une batterie 25 et permet de transmettre une information d'activation à un circuit de contrôle et de commande 24. Le circuit de contrôle et de commande 24 commande un vérin 27 comportant deux chambres 28, 29 asservies par une servovalve 30. La servovalve 30 et le circuit de contrôle et de commande 24 sont également alimentés par la batterie 25. Le vérin 27 est associé à un embrayage 31 permettant de lier les déplacements du vérin 27 soit à une vis 14 ou à deux pointes 15a, 15b. L'embrayage 31 est également commandé par le circuit de contrôle et de commande 24.

Les efforts de déplacement du vérin 27 sont mesurés par deux capteurs 17, 19. Un premier capteur 17 est apte à mesurer les efforts de pénétration Ev de la vis 14 dans le poteau en bois 11 et un deuxième capteur 19 est apte à mesurer les efforts de pénétration Ep des deux pointes 15a, 15b dans le poteau en bois 11. Un troisième capteur 18 permet de mesurer une résistance électrique Re du poteau en bois 11 entre les deux extrémités des deux pointes 15a, 15b lorsqu'elles sont enfoncées dans le poteau en bois 11. Ces trois mesures sont transmises au circuit de contrôle et de commande 24 qui affiche ces mesures sur un afficheur 32.

De préférence, le dispositif 10 comporte une pièce de guidage 33 comportant deux pointes 34a, 34b fixes par rapport cadre 12 disposées de part et d'autre du dispositif 10 au niveau de l'extrémité avant 20.

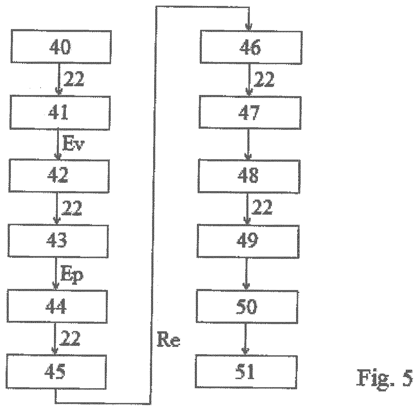

La Figure 5 illustre les étapes d'un procédé de métrologie de la qualité du poteau en bois 11 mises en oeuvre par un opérateur. Dans une première étape 40, l'opérateur met en place le dispositif 10 contre le poteau 11 tel que représenté sur les Figures 1 et 2. Les deux pointes 34a, 34b de la pièce de guidage 33 sont insérées de quelques millimètres dans le poteau 11 de sorte à centrer l'orientation du dispositif 10 par rapport à une droite X passant sensiblement par le diamètre du poteau 11. En outre, le dispositif 10 est incliné par rapport au sol S avec un angle α compris entre 30 et 45 degrés. Au cours de cette étape 40, l'opérateur effectue une estimation de la position du dispositif 10 par rapport au poteau 11 à l'oeil nu. En variante, le dispositif 10 peut comporter des moyens pour améliorer la détection de cette position (laser, réglet ...).

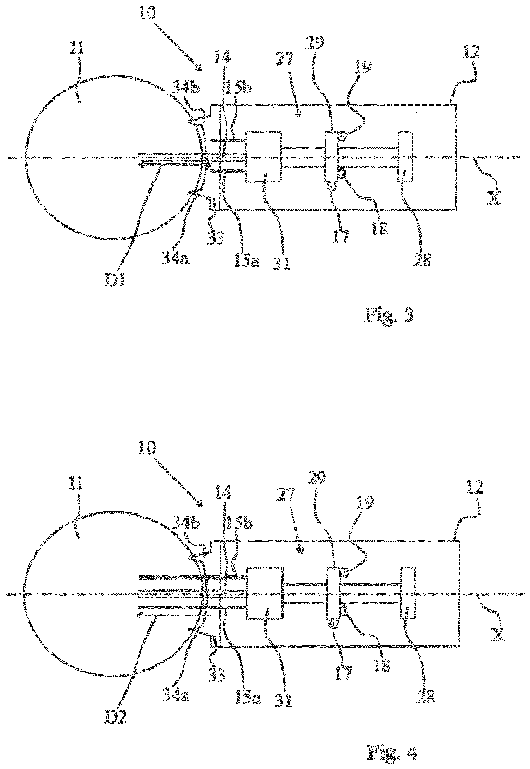

Lorsque le dispositif 10 est correctement mis en place sur le poteau 11, l'opérateur actionne la gâchette 22. Dans une étape 41, le circuit 24 commande alors l'embrayage 31 pour associer les déplacements du vérin 27 avec la vis 14. Le circuit 24 actionne également le vérin 27 de sorte que la vis 14 pénètre dans le poteau 11 sur une distance prédéterminée D1 tel que représenté sur la Figure 3. La pénétration de la vis 14 est effectuée en suivant l'angle α par rapport au sol S et la vis 14 s'étend dans le poteau 11 sous le niveau du sol S. Au cours des déplacements de la vis 14 jusqu'à la distance prédéterminée D1, le capteur 17 mesure les efforts du vérin 27. La distance D1 est préférentiellement comprise entre 40 et 60 mm. Cette mesure Ev est transmise au circuit 24 qui affiche cette mesure Ev sur l'afficheur 32 pour l'opérateur dans une étape 42. A partir de cette étape 42, la vis 14 assure le maintien du dispositif 10 avec le poteau 11.

L'opérateur actionne ensuite la gâchette 22 et, dans une étape 43, le circuit 24 commande l'embrayage 31 pour associer les déplacements du vérin 27 avec les deux pointes 15a, 15b. Le circuit 24 actionne également le vérin 27 de sorte que les deux pointes 15a, 15b pénétrèrent l'une après l'autre dans le poteau 11 sur une distance prédéterminée D2 tel que représenté sur la Figure 4. La distance D2 est préférentiellement comprise entre 40 et 60 mm. En variante, les deux pointes 15a, 15b pénétrèrent en même temps dans le poteau 11.

La pénétration de chaque pointe 15a, 15b est effectuée en suivant l'angle α par rapport au sol S et chaque pointe 15a, 15b s'étend dans le poteau 11 sous le niveau du sol S. L'angle de la vis 14 par rapport au cadre 12 étant identique à l'angle des pointes 15a, 15b par rapport au cadre 12, la force exercée sur le cadre 12 lors de l'étape 43 a une direction opposée à la force de maintien du cadre 12 par la vis 14. Au cours des déplacements des deux pointes 15a, 15b jusqu'à la distance prédéterminée D2, le capteur 19 mesure les efforts du vérin 27 pour l'enfoncement de chaque pointe 15a, 15b. Ces deux mesures Ep sont transmises au circuit 24 qui affiche ces mesures Ep sur l'afficheur 32 pour l'opérateur dans une étape 44.

L'opérateur actionne une nouvelle fois la gâchette 22 lorsqu'il a pris connaissance des mesures Ep et, dans une étape 45, le circuit 24 interroge le capteur 18 pour mesurer une résistivité électrique Re entre les extrémités des deux pointes 15a, 15b qui sont insérées dans le poteau 11. Cette mesure Re est transmise au circuit 24 qui affiche cette mesure Re sur l'afficheur 32 pour l'opérateur dans une étape 46.

Les mesures étant terminées, l'opérateur actionne la gâchette 22 pour que, dans une étape 47, le circuit 24 actionne le vérin 27 afin de retirer les pointes 15a, 15b du poteau 11. Lorsque les pointes 15a, 15b ont retrouvées leurs positions initiales, le circuit 24 informe l'opérateur sur l'afficheur 32 dans une étape 48. L'opérateur actionne une dernière fois la gâchette 22 pour que, dans une étape 49, le circuit 24 commande l'embrayage 31 pour associer les déplacements du vérin 27 avec la vis 14. Le circuit 24 actionne également le vérin 27 de sorte que la vis 14 retrouve sa position initiale. Lorsque la vis 14 a atteint sa position initiale, le circuit 24 en informe l'opérateur dans une étape 50 au moyen de l'afficheur 32. A partir de l'étape 50, la vis 14 n'assure plus le maintien du dispositif 10 sur le poteau 11. La dernière étape 51 consiste à extraire les deux pointes 34a, 34b du poteau 11 pour retirer complètement le dispositif 10.

De préférence, les déplacements du vérin 27 sont régulés par la servovalve 30 commandée par le circuit 24 lors des étapes 41, 43 de déplacement de la vis 14 et/ou des pointes 15a, 15b dans le poteau 11. Les pointes 15a, 15b et/ou la vis 14 peuvent également être montés sur des éléments de fixation amovible par rapport au cadre 12 de sorte à faciliter le remplacement de ces outils. La vis 14, par exemple, peut être changée par l'opérateur en fonction de l'essence et du diamètre du poteau 11.

Le mode de réalisation décrit un affichage des mesures Ev, Ep et Er directement sur l'afficheur 32 pour l'opérateur. L'opérateur peut ainsi estimer un état de sécurité et une durée de vie résiduelle du poteau 11 en fonction des mesures Ev, Ep et Er. En variante, le circuit 24 comporte des moyens de stockage des mesures Ev, Ep et Er et des moyens de traitement aptes à estimer un état de sécurité et une durée de vie résiduelle du poteau 11. Dans une autre variante, le circuit 24 comporte des moyens d'émission sans fil des mesures Ev, Ep et Er et un calculateur distant réceptionne ces mesures Ev, Ep et Er et réalise l'analyse. En plus des mesures Ev, Ep et Er d'autres informations peuvent également être prises en considération, pour estimer un état de sécurité et une durée de vie résiduelle, telles que l'essence du poteau 11, le diamètre, et le type de traitement chimique subit par le poteau 11.

L'invention permet ainsi de tester la qualité d'un poteau 11 et d'estimer un état de sécurité et une durée de vie résiduelle. En variante, l'invention peut être utilisée pour caractérisée tout type de matériau hygroscopique, tel que du bois ou autres matériaux de construction analogues.

## Revendications

1. Dispositif de métrologie (10) de la qualité d'un matériau hygroscopique (11), tel que du bois ou autres matériaux de construction analogues, comprenant :
- un cadre (12),
- un élément de support (14) apte à fixer le cadre (12) au matériau hygroscopique (11),
- deux pointes (15a, 15b) mobiles en translation par rapport au cadre (12),
- des moyens de mesure (17) d'un effort (Ep) de pénétration de chaque pointe (15a, 15b) en fonction d'une force appliquée sur chaque pointe (15a, 15b) pour atteindre une distance prédéterminée,
- des moyens de mesure (18) d'une résistivité électrique (Re) entre les extrémités des deux pointes (15a, 15b) aptes à mesurer une résistivité électrique (Re) du matériau hygroscopique (11) lorsque les deux pointes (15a, 15b) sont insérées dans le matériau hygroscopique (11),
**caractérisé en ce que**, l'élément de support (14) étant apte à pénétrer dans le matériau hygroscopique (11) pour fixer le cadre (12) au matériau hygroscopique (11), le dispositif comporte également :
- des moyens de mesure (19) d'un effort (Ev) de pénétration de l'élément de support (14) en fonction d'une force appliquée sur l'élément de support (14) pour atteindre une position de fixation prédéterminée.

2. Dispositif de métrologie selon la revendication 1, **caractérisé en ce que** le dispositif comporte :
- au moins un moyen de déplacement motorisé des pointes (15a, 15b) et/ou de l'élément de support (14), ledit moyen de déplacement étant commandé par une commande de déplacement, et
- au moins un moyen de régulation de la commande de déplacement.

3. Dispositif de métrologie selon la revendication 1 ou 2, **caractérisé en ce que** le dispositif comporte au moins une source d'énergie autonome.

4. Dispositif de métrologie selon l'une des revendications 1 à 3, **caractérisé en ce que** le dispositif comporte une pièce de guidage (33) de la position du cadre (12) par rapport au matériau hygroscopique (11), la pièce de guidage (33) comportant deux pointes (34a, 34b) aptes à être insérées dans le matériau hygroscopique (11) sur quelques millimètres.

5. Dispositif de métrologie selon l'une des revendications 1 à 4, **caractérisé en ce que** les pointes (15a, 15b) et/ou l'élément de support (14) sont montés sur des éléments de fixation amovible par rapport au cadre (12).

6. Dispositif de métrologie selon l'une des revendications 1 à 5, **caractérisé en ce que** le dispositif comporte :
- des moyens de stockage des valeurs des efforts (Ep) de pénétration de chaque pointe (15a, 15b), de la résistivité électrique (Re) et de l'effort (Ev) de pénétration de l'élément de support (14), et
- des moyens de traitement aptes à estimer un état de sécurité et une durée de vie résiduelle du matériau hygroscopique (11) en fonction des valeurs des efforts (Ep) de pénétration de chaque pointe (15a, 15b), de la résistivité électrique (Re) et de l'effort (Ev) de pénétration de l'élément de support (14).

7. Dispositif de métrologie selon l'une des revendications 1 à 5, **caractérisé en ce que** le dispositif comporte :
- des moyens d'émission disposés à l'intérieur du cadre (12) aptes à transmettre les valeurs des efforts (Ep) de pénétration de chaque pointe (15a, 15b), la résistivité électrique (Re) et l'effort (Ev) de pénétration de l'élément de support (14), et
- un calculateur de terrain distant par rapport au cadre (12) comportant :
∘ des moyens de réception aptes à recevoir les informations des moyens d'émission disposés à l'intérieur du cadre (12),
∘ des moyens de stockage des paramètres du matériau hygroscopique (11), et
∘ des moyens de traitement intégrés dans le calculateur de terrain aptes à estimer un état de sécurité et une durée de vie résiduelle du matériau hygroscopique (11) en fonction des valeurs des efforts (Ep) de pénétration de chaque pointe (15a, 15b), de la résistivité électrique (Re), de l'effort (Ev) de pénétration de l'élément de support (14), et des paramètres du matériau hygroscopique (11).

8. Dispositif de métrologie selon la revendication 7, **caractérisé en ce que** les paramètres du matériau hygroscopique (11) comportent l'essence du matériau hygroscopique (11), le diamètre, le type de traitement chimique subit par le matériau hygroscopique (11) et la longueur hors sol du matériau hygroscopique (11).

9. Dispositif de métrologie selon l'une des revendications 1 à 8, **caractérisé en ce que** la distance prédéterminée d'enfoncement des pointes (15a, 15b) est comprise entre 40 et 60mm.

10. Dispositif de métrologie selon l'une des revendications 1 à 9, **caractérisé en ce que** l'élément de support (14) est une vis à bois dont le diamètre est adapté à l'essence et au diamètre du matériau hygroscopique (11).

11. Procédé de métrologie de la qualité d'un matériau hygroscopique (11), tel que du bois ou d'autres matériaux de construction analogues, comprenant les étapes consistant à :
- insérer deux pointes (15a, 15b) dans le matériau hygroscopique (11) jusqu'à une distance prédéterminée,
- mesurer, pour chaque pointe (15a, 15b), un effort (Ep) de pénétration en fonction de la force appliquée sur chaque pointe (15a, 15b) pour atteindre la distance prédéterminée,
- lorsque les deux pointes (15a, 15b) ont atteints la distance prédéterminée, mesurer une résistivité électrique (Re) entre les extrémités des deux pointes (15a, 15b) insérée dans le matériau hygroscopique (11),
- estimer une hygrométrie interne du matériau hygroscopique (11) en fonction de la résistivité électrique (Re),
**caractérisé en ce que** le procédé comporte les étapes consistant à :
- insérer un élément de support (14) dans le matériau hygroscopique (11) suivant un angle par rapport au cadre (12) sensiblement égal à l'angle de pénétration des pointes (15a, 15b) par rapport au cadre (12),
- mesurer un effort (Ev) de pénétration appliquée sur l'élément de support (14) pour atteindre une position de fixation prédéterminée, et
- estimer un état de sécurité et une durée de vie résiduelle du matériau hygroscopique (11) en fonction des valeurs des efforts (Ep) de pénétration de chaque pointe (15a, 15b), de la résistivité électrique (Re) et de l'effort (Ev) de pénétration de l'élément de support (14).

12. Procédé de métrologie selon la revendication 11, **caractérisé en ce que**, le matériau hygroscopique (11) étant planté sur un sol (S), l'étape consistant à insérer un élément de support (14) dans le matériau hygroscopique (11) est effectuée au niveau du sol (S).

13. Procédé de métrologie selon la revendication 11 ou 12, **caractérisé en ce que** l'élément de support (14) pénètre dans le matériau hygroscopique (11) suivant un angle (α) par rapport au sol (S) sensiblement égal à l'angle de pénétration des pointes (15a, 15b) par rapport au sol (S).

14. Procédé de métrologie selon la revendication 13, **caractérisé en ce que** les étapes consistant à insérer l'élément de support (14) et les deux pointes (15a, 15b) dans le matériau hygroscopique (11) sont effectuées avec un angle (α) compris entre 30 et 45 degrés par rapport au sol (S).

15. Procédé de métrologie selon l'une des revendications 11 à 14, **caractérisé en ce que**, le matériau hygroscopique (11) étant planté sur un sol, l'étape consistant à insérer un élément de support (14) dans le matériau hygroscopique (11) est effectuée avec un angle (α) compris entre 30 et 45 degrés par rapport au sol (S) de sorte que l'élément de support (14) pénètre sous le niveau du sol.

## Patentansprüche

1. Metrologievorrichtung (10) zur Bestimmung der Qualität eines hygroskopischen Materials (11), wie Holz oder analoge Baumaterialien, umfassend:
- einen Rahmen (12),
- ein Halteelement (14), mit dem der Rahmen (12) am hygroskopischen Material (11) befestigt werden kann,
- zwei Spitzen (15a, 15b), die in Bezug zum Rahmen verschoben werden können,
- Mittel zum Messen (17) einer Eindringkraft (Ep) jeder Spitze (15a, 15b), abhängig von einer auf jede Spitze (15a, 15b) ausgeübten Kraft, um eine vorher festgelegte Distanz zu erreichen,
- Mittel zum Messen (18) eines elektrischen Widerstands (Re) zwischen den Enden der beiden Spitzen (15a, 15b), die in der Lage sind, einen elektrischen Widerstand (Re) des hygroskopischen Materials (11) zu messen, wenn die beiden Spitzen (15a, 15b), in das hygroskopische Material (11) eingeführt werden. **dadurch gekennzeichnet, dass** das Halteelement (14) in der Lage ist, in das hygroskopische Material (11) einzudringen, um den Rahmen (12) am hygroskopischen Material (11) zu befestigen, wobei die Vorrichtung außerdem enthält:
- Mittel (19) zum Messen einer Eindringkraft (Ev) des Halteelementes (14) in Abhängigkeit von einer Kraft, die auf das Halteelement (14) ausgeübt wird, um eine vorher festgelegte Befestigungsposition zu erreichen.

2. Metrologievorrichtung gemäß Anspruch 1, **dadurch gekennzeichnet, dass** sie enthält:
- mindestens eine Vorrichtung zur motorgestützten Verlagerung der Spitzen (15a, 15b) und/ oder des Halteelementes (14), wobei diese Verlagerungsvorrichtung von einer Verlagerungssteuerung gesteuert wird, und
- mindestens eine Regelvorrichtung für die Verlagerungssteuerung.

3. Metrologievorrichtung gemäß Anspruch 1 oder 2, **dadurch gekennzeichnet, dass** die Vorrichtung mindestens eine autonome Energiequelle enthält.

4. Metrologievorrichtung gemäß einem der vorstehenden Ansprüche 1 bis 3, **dadurch gekennzeichnet, dass** die Vorrichtung ein Führungsteil (33) für die Position des Rahmens (12) bezogen auf das hygroskopische Material (11) enthält, das Führungsteil (33) dabei zwei Spitzen (34a, 34b) enthält, die in das hygroskopische Material (11) einige Millimeter tief eingeführt werden können.

5. Metrologievorrichtung gemäß einem der vorstehenden Ansprüche 1 bis 4, **dadurch gekennzeichnet, dass** die Spitzen (15a, 15b) und/ oder das Halteelement (14) auf Elemente montiert sind, die bezogen auf den Rahmen (12) beweglich sind

6. Metrologievorrichtung gemäß einem der vorstehenden Ansprüche 1 bis 5, **dadurch gekennzeichnet, dass** die Vorrichtung außerdem umfasst:
- Mittel zur Speicherung der Werte der Eindringkräfte (Ep) jeder Spitze (15a, 15b), des elektrischen Widerstandes (Re) und der Eindringkraft (Ev) des Halteelementes (14), und
- Behandlungsmittel, die in der Lage sind, einen Sicherheitszustand und eine Restlebensdauer des hygroskopischen Materials (11) entsprechend der Eindringkraft (Ep) jeder Spitze (15a, 15b), dem elektrischen Widerstand (Re) und der Eindringkraft (Ev) des Halteelementes (14) zu schätzen.

7. Metrologievorrichtung gemäß einem der vorstehenden Ansprüche 1 bis 5, **dadurch gekennzeichnet, dass** die Vorrichtung außerdem umfasst:
- Emissionsvorrichtungen, die im Inneren des Rahmens (12) angeordnet ist und die in der Lage sind, die Werte der Eindringkräfte (Ep) jeder Spitze (15a, 15b), des elektrischen Widerstandes (Re) und der Eindringkraft (Ev) des Halteelementes (14) zu übertragen, und
- einen Geländerechner, die vom Rahmen (12) entfernt sind, der umfasst:
∘ Empfangsvorrichtungen zum Empfang von Informationen der im Innern des Rahmens (12) angeordneten Emissionsvorrichtungen,
∘ Mittel zur Speicherung der Parameter des hygroskopischen Materials (11), und
∘ Behandlungsmittel, die in den Geländerechner integriert sind und die in der Lage sind, einen Sicherheitszustand und eine Restlebensdauer des hygroskopischen Materials (11) entsprechend den Werten der Eindringkraft (Ep) jeder Spitze (15a, 15b), dem elektrischen Widerstand (Re), der Eindringkraft (Ev) des Halteelementes (14) und der Parameter des hygroskopischen Materials (11) zu schätzen.

8. Metrologievorrichtung gemäß Anspruch 7, **dadurch gekennzeichnet, dass** die Parameter des hygroskopischen Materials (11) die Art des hygroskopischen Materials (11), den Durchmesser, die Art der chemischen Behandlung des hygroskopischen Materials (11) und die Länge über Boden des hygroskopischen Materials (11) enthalten.

9. Metrologievorrichtung gemäß einem der vorstehenden Ansprüche 1 bis 8, **dadurch gekennzeichnet, dass** die vorher festgelegte Eindringtiefe der Spitzen (15a, 15b) zwischen 40 und 60mm liegt.

10. Metrologievorrichtung gemäß einem der vorstehenden Ansprüche 1 bis 9, **dadurch gekennzeichnet, dass** das Halteelement (14) eine Holzschraube ist, deren Durchmesser an die Art und den Durchmesser des hyroskopischen Materials (11) angepasst ist.

11. Messverfahren für die Qualität eines hygroskopischen Materials (11), wie Holz oder analoge Baumaterialien, das die folgenden Schritte umfasst:
- zwei Spitzen (15a, 15b) bis zu einer vorher festgelegten Tiefe in das hygroskopische Material (11) einführen,
- für jede Spitze (15a, 15b), eine Eindringkraft (Ep), entsprechend der auf jede Spitze (15a, 15b) ausgeübten Kraft, um die vorher bestimmte Distanz zu erreichen, messen,
- wenn die beiden Spitzen (15a, 15b) die vorher festgelegte Distanz erreicht haben, den elektrischen Widerstand (Re) zwischen den Enden der beiden Spitzen (15a, 15b), die in das hygroskopische Material (11) eingeführt wurden, messen,
- innere Hygrometrie des hygroskopischen Materials (11) entsprechend dem elektrischen Widerstand (Re) schätzen,
**dadurch gekennzeichnet, dass** das Verfahren die folgenden Schritte umfasst:
- ein Halteelement (14) in das hygroskopische Material (11) entsprechend einem Winkel, bezogen auf den Rahmen (12) einführen, der im Wesentlichen gleich dem Eindringwinkel der Spitzen (15a, 15b) bezogen auf den Rahmen (12) ist,
- Messung einer Eindringkraft (Ev), angewandt auf das Halteelement (14), um eine vorher festgelegte Befestigungsposition zu erreichen, und
- Sicherheitszustand und Restlebensdauer des hygroskopischen Materials (11) entsprechend der Eindringkraft (Ep) jeder Spitzen (15a, 15b), dem elektrischen Widerstand (Re) und der Eindringkraft (Ev) des Halteelementes (14) schätzen.

12. Messverfahren nach Anspruch 11, **dadurch gekennzeichnet, dass** das hygroskopische Material (11) auf einem Boden (S) steht, wobei der Schritt, der darin besteht, ein Halteelement (14) in das hygroskopische Material (11) einzuführen, auf Bodenhöhe (S) durchgeführt wird.

13. Messverfahren nach Anspruch 11 oder 12, **dadurch gekennzeichnet, dass** das Halteelement (14) in das hygroskopische Material (11) entsprechend einem auf den Boden (S) bezogenen Winkel (a) eingeführt wird, der im Wesentlichen gleich dem Eindringwinkel der Spitzen (15a, 15b) bezogen auf den Boden (S) ist,

14. Messverfahren nach Anspruch 13, **dadurch gekennzeichnet, dass** der Schritt, der darin besteht, das Halteelement (14) und die beiden Spitzen (15a, 15b) in das hygroskopische Material (11) einzuführen, mit einem Winkel (a) zwischen 30 und 45 Grad bezogen auf den Boden (S) durchgeführt werden.

15. Messverfahren nach einem der vorstehenden Ansprüche 11 bis 14, **dadurch gekennzeichnet, dass** wenn das hygroskopische Material (11) auf einem Boden steht, der Schritt, der darin besteht, das Halteelement (14) in das hygroskopische Material (11) einzuführen, mit einem Winkel (a) zwischen 30 und 45 Grad bezogen auf den Boden (S) durchgeführt wird, so dass das Halteelement (14) unter Bodenniveau eindringt.

## Claims

1. Metrology device (10) for determining the quality of a hygroscopic material (11), such as wood or other similar construction materials, comprising:
- a frame (12),
- a support element (14) suitable for attaching the frame (12) to the hygroscopic material (11),
- two tips (15a, 15b) that can move in translation with respect to the frame (12),
- means (17) for measuring a penetration effort (Ep) of each tip (15a, 15b) as a function of a force applied to each tip (15a, 15b) in order to reach a predetermined distance,
- means (18) of measuring an electrical resistivity (Re) between the ends of the two tips (15a, 15b), which means are capable of measuring an electrical resistivity of the hygroscopic material (11) when the two tips (15a, 15b) are inserted into the hygroscopic material (11),
**characterized in that**, the support element (14) being capable of penetrating into the hygroscopic material (11) in order to fix the frame (12) to the hygroscopic material (11), the device also comprises:
- means (19) of measuring a penetration effort (Ev) of the support element (14) as a function of a force applied to the support element (14) to reach a predetermined fixing position.

2. Metrology device according to claim 1, **characterized in that** the device comprises:
- at least one motorized means of moving tips (15a, 15b) and/or the support element (14), said movement means being controlled by a movement control, and
- at least one means of regulating the control of movement.

3. Metrology device according to either of claims 1 or 2, **characterized in that** the device comprises at least one independent energy source.

4. Metrology device according to any one of claims 1 to 3, **characterized in that** the device comprises a part (33) to guide the position of the frame (12) with respect to the hygroscopic material (11), the guide part (33) comprising two tips (34a, 34b) capable of being inserted several millimeters into the hygroscopic material (11).

5. Metrology device according to any one of claims 1 to 4, **characterized in that** the tips (15a, 15b) and/or the support element (14) are mounted on fixing elements removable with respect to the frame (12).

6. Metrology device according to any one of claims 1 to 5, **characterized in that** the device comprises:
- means of storing values for the penetration effort (Ep) of each tip (15a, 15b), for the electrical resistivity (Re) and for the penetration effort (Ev) of the support element (14), and
- processing means capable of estimating the safety status and remaining working life of the hygroscopic material (11) as a function of the values for the penetration efforts (Ep) of each tip (15a, 15b), for the electrical resistivity (Re) and for the penetration effort (Ev) of the support element (14).

7. Metrology device according to any one of claims 1 to 5, **characterized in that** the device comprises:
- transmission means located inside the frame (12) capable of transmitting the values for the penetration efforts (Ep) of each tip (15a, 15b), for the electrical resistivity (Re) and for the penetration effort (Ev) of the support element (14), and
- a field computer, remote with respect to the frame (12), comprising:
∘ reception means capable of receiving the information from the transmission means located inside the frame (12),
∘ means of storing parameters of the hygroscopic material (11), and
∘ processing means integrated into the field computer capable of estimating the safety status and remaining working life of the hygroscopic material (11) as a function of the values for the penetration efforts (Ep) of each tip (15a, 15b), for the electrical resistivity (Re) and for the penetration effort (Ev) of the support element (14), and for the parameters of the hygroscopic material (11).

8. Metrology device according to claim 7, **characterized in that** the parameters of the hygroscopic material (11) comprise the substance of the hygroscopic material (11), the diameter, type of chemical processing undergone by the hygroscopic material (11), and the above-ground length of the hygroscopic material (11).

9. Metrology device according to any one of claims 1 to 8, **characterized in that** the predetermined distance of insertion of the tips (15a, 15b) is between 40 and 60 mm.

10. Metrology device according to any one of claims 1 to 9, **characterized in that** the support element (14) is a wood screw the diameter whereof is adapted to the substance and diameter of the hygroscopic material (11).

11. Metrology method for determining the quality of a hygroscopic material (11), such as wood or other similar construction materials, comprising the steps consisting of:
- inserting two tips (15a, 15b) into the hygroscopic material (11) up to a predetermined distance,
- measuring, for each tip (15a, 15b), a penetration effort (Ep) as a function of the force applied to each tip (15a, 15b) to reach a predetermined distance,
- when the two tips (15a, 15b) have reached the predetermined distance, measuring an electrical resistivity (Re) between the ends of the two tips (15a, 15b) inserted into the hygroscopic material (11),
- estimating an internal hygrometry of the hygroscopic material (11) as a function of the electrical resistivity (Re),
**characterized in that** the method comprises the steps consisting of:
- inserting a support element (14) into the hygroscopic material (11) at an angle with respect to the frame (12) that is substantially equal to the angle of penetration of the tips (15a, 15b) with respect to the frame (12),
- measuring a penetration effort (Ev) applied to the support element (14) in order to reach a predetermined fixing position, and
- estimating the safety status and remaining working life of the hygroscopic material (11) based on the values for the penetration efforts (Ep) of each tip (15a, 15b), for the electrical resistivity (Re) and for the penetration effort (Ev) of the support element (14).

12. Metrology method according to claim 11, **characterized in that**, the hygroscopic material (11) being planted in the ground (S), the step consisting of inserting a support element (14) in the hygroscopic material (11) is performed at ground level (S).

13. Metrology method according to claim 11 or 12, **characterized in that** the support element (14) penetrates into the hygroscopic material (11) at an angle (α) with respect to the ground (S) substantially equal to the angle of penetration of the tips (15a, 15b) with respect to the ground (S).

14. Metrology method according to claim 13, **characterized in that** the steps consisting of inserting the support element (14) and the two tips (15a, 15b) into the hygroscopic material (11) are performed at an angle (α) of between 30 and 45 degrees with respect to the ground (S).

15. Metrology method according to any one of claims 11 to 14, **characterized in that**, the hygroscopic material (11) being planted in the ground, the step consisting of inserting a support element (14) in the hygroscopic material (11) is performed at an angle (α) of between 30 and 45 degrees with respect to the ground (S) such that the support element (14) penetrates beneath ground level.
